# FASCICULE DE BREVET EUROPEEN

(11) **EP 1 870 082 B1**
(45) Date de publication et mention de la délivrance du brevet: **01.05.2013**
(21) Numéro de dépôt: 07110785.8
(22) Date de dépôt: 21.06.2007
(51) Int. Cl.: A61K 8/34, A61Q 1/04, A61Q 1/10, A61Q 3/02, A61K 8/85, A61K 8/36, A61K 8/362, A61Q 1/06

(54) **Composition cosmétique comprenant un polycondensat**
Kosmetische Zusammensetzung enthaltend ein Polykondensat
Cosmetic composition comprising a condensation polymer

(30) Priorité: 22.06.2006 FR 0652591
(43) Date de publication de la demande: 26.12.2007
(73) Titulaire: L'Oréal, 75008 Paris (FR)
(72) Inventeur: Malle, Gérard, 77580, Villiers S/Morin (FR); Mondet, Jean, 93600, Aulnay Sous Bois (FR); Blin, Xavier, 75015, Paris (FR); Rodriguez, Ivan, 60290, Cauffry (FR); Giustiniani, Pascal, 92250 La Garenne Colombes (FR)
(74) Mandataire: Kromer, Christophe

(56) Documents cités:
- US-A- 2 915 488
- DATABASE WPI Week 198808 Thomson Scientific, London, GB; AN 1988-052800 XP002417277 & JP 63 008318 A (KOBAYASHI KOSE KK) 14 janvier 1988 (1988-01-14)
- DATABASE WPI Week 197814 Thomson Scientific, London, GB; AN 1978-26140A XP002409236 & JP 53 018742 A (KANEBO LTD) 21 février 1978 (1978-02-21)
- DATABASE WPI Week 198312 Thomson Scientific, London, GB; AN 1983-28350K XP002417278 & JP 58 023614 A (ASANUMA KOGYO KK) 12 février 1983 (1983-02-12)
- DATABASE WPI Week 197910 Thomson Scientific, London, GB; AN 1979-18769B XP002423623 & JP 54 011244 A (KANEBO LTD) 27 janvier 1979 (1979-01-27)

## Description

La présente invention a trait à de nouveaux polymères de la famille des polycondensats de type alkyde modifié, ainsi qu'à leur utilisation dans les compositions cosmétiques, notamment dans les rouges à lèvres, aux compositions cosmétiques les contenant et aux procédés de préparation desdits polycondensats.

Il existe de nombreuses compositions cosmétiques pour lesquelles des propriétés de brillance du film déposé, après application sur les matières kératiniques (peau, lèvres, phanères), sont souhaitées. On peut citer par exemple les rouges à lèvres, les vernis à ongles ou encore certains produits capillaires.
Afin d'obtenir un tel résultat, il est possible d'associer des matières premières particulières, notamment des lanolines, avec des huiles dites brillantes, telles que les polybutènes qui présentent toutefois une viscosité élevée; ou des esters d'acide
ou d'alcool gras dont le nombre de carbone est élevé; ou bien certaines huiles végétales; ou encore des esters résultants de l'estérification partielle ou totale d'un composé aliphatique hydroxylé avec un acide aromatique, comme décrit dans la demande de brevet EP1097699.
Il est également connu d'associer des lanolines avec des polyesters obtenus par réaction séquencée de l'huile de ricin avec l'acide isostéarique puis avec l'acide succinique, tel que décrit dans le brevet US6342527.
Pour améliorer la brillance du film déposé, ainsi que sa tenue, il a également été proposé d'utiliser des esters résultant de la condensation d'un polyol avec un acide carboxylique de type "néo", notamment dans FR2838049.
On peut également citer EP1457201, qui décrit une composition associant un polyester de triglycérides d'acides carboxyliques hydroxylés et une huile de faible masse moléculaire choisie parmi les polybutylènes, les polyisobutylènes hydrogénés, les polydécènes hydrogénés ou non, les copolymères de vinylpyrrolidones, les esters d'acides gras linéaires, les esters hydroxylés, les esters d'alcools gras
ou d'acides gras ramifiés en C24-C28, les huiles siliconées et/ou les huiles d'origine végétale.
Dans la demande de brevet EP0792637, il est décrit une composition associant un ester aromatique et un polymère de type polybutène ou polyisobutène.
Dans la demande de brevet EP1155687, on décrit un procédé consistant à incorporer, dans une phase huileuse constituée d'une huile cosmétiquement acceptable, un organopolysiloxane possédant au moins 2 groupes susceptibles d'établir des liaisons hydrogènes.
Toutefois, ces compositions et associations, même si elles améliorent la brillance de manière significative, sont encore jugées insuffisantes dans une optique de longue tenue de cette brillance, dans le temps.

Le but de la présente invention est de proposer de nouveaux polymères qui peuvent conférer une brillance significative à un dépôt notamment filmogène, tout en maintenant une bonne durabilité dans le temps de cette brillance; ceci peut trouver une application particulièrement avantageuse dans le domaine des rouges à lèvres. Par ailleurs, on recherche également des polymères peuvent en outre avantageusement conférer à la composition une excellente tenue dans le temps sur les matières kératiniques, notamment sur les lèvres.

A cette fin, la demanderesse a recherché de nouveaux polycondensats de type alkyde, ayant les propriétés recherchées.
Les résines alkydes constituent une classe particulière de polyesters en étant le produit de réaction de polyols et d'acides polycarboxyliques, généralement modifié par des acides gras insaturés, tels que l'acide oléique, ou par des huiles insaturées, huile de soja ou de ricin par exemple, qui permettent de moduler leurs propriétés filmogènes, notamment leur vitesse de séchage, leur dureté, leur résistance.
Ainsi, il a été proposé dans le document US2915488 des résines alkydes modifiées dans lesquelles une partie des acides gras provenant de l'huile de soja a été remplacée par de l'acide benzoïque. Ces nouvelles résines présentent des propriétés améliorées en terme de résistance aux alcalis et aux détergents; les films les contenant sèchent plus rapidement et sont plus durs. Toutefois, aucune application, notamment cosmétique ou topique, n'a été envisagée pour ces résines.
Par ailleurs, les acides gras présents dans l'huile de soja sont majoritairement constitués de deux acides gras insaturés: environ 55% d'acide linoléique (C18:2) et 28% d'acide oléique (C18:1), selon "Surface Coatings Science and Technology", 2ème édition, JOHN WILEY & Sons, pages 104 et 105. Or, on sait que certains acides gras insaturés peuvent subir, au cours du temps, une autooxydation qui peut être à l'origine de phénomènes de rancissement, qui peuvent déboucher sur des problèmes de conservation des compositions comprenant ces matières premières. De plus, les résines alkydes décrites dans US2915488, qui comprennent une proportion élevée d'acides gras linoléique et oléique, ne sont pas optimales, notamment en terme de stabilité, pour une utilisation en cosmétique.

La réticulation des résines alkydes par oxydation à l'air avec formation d'hydroperoxydes joue un rôle clé dans la vitesse de séchage du film, et, par suite, dans ses propriétés finales de dureté et de résistance aux agressions extérieures. Dans le domaines habituel d'application de ces résines alkydes, à savoir les peintures, la vitesse de réticulation, et donc de séchage, est généralement accélérée par addition de sels de métaux particuliers, dénommés "driers", tels que les naphténates et octanoates de cobalt qui accélèrent la décomposition des hydroperoxydes; ceci est notamment décrit dans Principles of Polymerization, 4ème édition, JOHN WILEY & SONS, pages 737-738 et également dans Surface Coatings Science and Technology, 2ème édition, JOHN WILEY & SONS, tables 2.3 et 2.4 pages 526-530. Toutefois, l'utilisation de tels sels métalliques n'est bien évidemment pas souhaitable en cosmétique pour des raisons évidentes de toxicité.

Par ailleurs, la plupart des résines alkydes n'a pas une solubilité convenable dans les milieux huileux habituellement utilisés en cosmétique, tels que les huiles végétales, les alcanes, les esters gras, les alcools gras, les huiles de silicone, et notamment comprenant l'isododécane, le Parléam, l'isononanoate d'isononyle, l'octyldodécanol, la phényl triméthicone, le benzoate d'alkyle en C12-C15 et/ou la D5 (décaméthylcyclopentasiloxane).

Après d'importantes recherches, la demanderesse a découvert de façon surprenante et inattendue que certains polycondensats à plus haute teneur en acides carboxyliques particuliers, dont les acides monocarboxyliques non aromatiques, pouvaient conduire à des performances améliorées en terme de brillance, de maintien de ladite brillance, et en outre de longue tenue du film obtenu, tout en étant véhiculables dans les milieux cosmétiques usuels, notamment les milieux huileux cosmétiques usuels.

La présente invention a donc pour objet une composition cosmétique ou pharmaceutique comprenant, dans un milieu cosmétiquement ou pharmaceutiquement acceptable, au moins un polycondensat susceptible d'être obtenu par réaction :
- de 10 à 30% en poids, par rapport au poids total du polycondensat, de pentaérythritol;
- de 30 à 80% en poids, par rapport au poids total du polycondensat, d'au moins un acide monocarboxylique non aromatique, saturé ou insaturé, linéaire, ramifié et/ou cyclique, comprenant 6 à 32 atomes de carbone, choisi parmi l'acide isostéarique seul ou l'acide stéarique seul
- de 0,1 à 10 % en poids, par rapport au poids total du polycondensat, d'acide benzoïque;
- de 5 à 40% en poids, par rapport au poids total du polycondensat, d'acide isophtalique.

Le rapport entre le nombre de mole d'acide monocarboxylique aromatique et le nombre de mole d'acide monocarboxylique non aromatique est compris entre 0,08 et 0,70.

Un autre objet de l'invention est un polycondensat susceptible d'être obtenu par réaction :
- de 10 à 30% en poids, par rapport au poids total du polycondensat, de pentaérythritol
- de 45 à 80% en poids, par rapport au poids total du polycondensat, d'au moins un acide monocarboxylique non aromatique saturé, linéaire, ramifié et/ou cyclique, comprenant 6 à 32 atomes de carbone choisi parmi l'acide isostéarique seul ou l'acide stéarique seul
- de 0,1 à 10% en poids, par rapport au poids total du polycondensat, d'acide benzoïque;
- de 5 à 40% en poids, par rapport au poids total du polycondensat, d'acide isophtalique.

Notamment, les compositions cosmétiques présentent une bonne applicabilité et une bonne couvrance; une bonne adhérence sur le support, que cela soit sur l'ongle, les cheveux, les cils, la peau ou les lèvres; une flexibilité et une résistance du film adéquates, afin d'éviter les craquelures, par exemple dans le cas des vernis
ou des rouges à lèvres; ainsi qu'un excellent niveau de brillance durable.
Les propriétés de confort et de glissant sont également très satisfaisantes.
Ces polycondensats sont aisément véhiculables dans les milieux solvants ou huileux cosmétiques, notamment les huiles, les alcools gras et/ou les esters gras, ce qui facilite leur mise en oeuvre dans le domaine cosmétique, notamment dans les rouges à lèvres ou les fonds de teint.
Les polycondensats selon l'invention peuvent être préparés aisément, en une seule étape de synthèse, et sans produire de déchets, ceci à faible coût.
Par ailleurs, il est aisément possible de modifier la structure et/ou les propriétés des polycondensats selon l'invention, en faisant varier la nature chimique des différents constituants et/ou leurs proportions.

Les polycondensats selon l'invention sont avantageusement branchés (ramifiés); on peut penser que ceci permet de générer un réseau par enchevêtrement des chaînes polymériques, et donc d'obtenir les propriétés recherchées, notamment en terme de tenue améliorée, de brillance améliorée, et en terme de solubilité. On a en effet constaté que les polycondensats linéaires ne permettaient pas d'obtenir une amélioration notable de la tenue de la composition, et que les polycondensats de type dendrimères, dont les chaînes sont régulières, ne présentaient pas une solubilité optimale.

Les polycondensats selon l'invention sont des polycondensats de type alkyde, et sont donc susceptibles d'être obtenus par estérification/polycondensation, selon les méthodes connues de l'homme du métier.

Le polycondensat selon l'invention peut en outre comprendre une silicone à fonction hydroxyle (OH) et/ou carboxylique (COOH).
Elle peut comprendre 1 à 3 fonctions hydroxyle et/ou carboxylique, et comprend de préférence deux fonctions hydroxyle ou bien deux fonctions carboxyliques. Ces fonctions peuvent être situées en bout de chaîne ou dans la chaîne, mais avantageusement en bout de chaîne.
On emploie de préférence des silicones ayant une masse moléculaire moyenne en poids (Mw) comprise entre 300 et 20000, notamment 400 et 10 000, voire 800 et 4000.
Cette silicone peut être de formule : 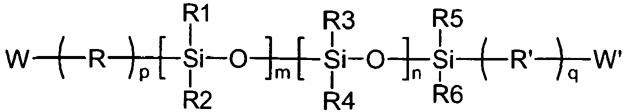 dans laquelle :
- W et W' sont, indépendamment l'un de l'autre, OH ou COOH; de préférence W=W';
- p et q sont, indépendamment l'un de l'autre, égaux à 0 ou 1,
- R et R' sont, indépendamment l'un de l'autre, un radical divalent carboné, notamment hydrocarboné, saturé ou insaturé, voire aromatique, linéaire, ramifié et/ou cyclique; comprenant 1 à 12 atomes de carbone, notamment 2 à 8 atomes de carbone, et comprenant éventuellement en outre 1 ou plusieurs hétéroatomes choisis parmi O, S et N, notamment O (éther);
   notamment R et/ou R' peuvent être de formule -(CH₂)ₐ- avec a=1-12, et notamment méthylène, éthylène, propylène, phénylène;
   ou bien de formule -[(CH₂)ₓ O)_{z}- avec x = 1, 2 ou 3 et z = 1-10; en particulier x=2 ou 3 et z=1-4; et mieux x=3 et z=1.
- R1 à R6 sont, indépendamment l'un de l'autre, un radical carboné linéaire, ramifié et/ou cyclique, saturé ou insaturé voire aromatique; comprenant 1 à 20 atomes de carbone, notamment 2 à 12 atomes de carbone; de préférence, R1 à R6 sont saturés ou bien aromatiques, et peuvent notamment être choisis parmi les radicaux alkyles, en particulier les radicaux méthyle, éthyle, propyle, isopropyle, butyle, pentyle, hexyle, octyle, décyle, dodécyle et octadécyle, les radicaux cycloalkyles, en particulier le radical cyclohexyle, les radicaux aryles, notamment phényle et naphtyle, les radicaux arylalkyles, notamment benzyle et phényléthyle, ainsi que les radicaux tolyle et xylyle.
- m et n sont, indépendamment l'un de l'autre, des entiers compris entre 1 et 140, et sont tels que la masse moléculaire moyenne en poids (Mw) de la silicone est comprise entre 300 et 20 000, notamment entre 400 et 10 000, voire entre 800 et 4000.
On peut notamment citer les polyalkylsiloxanes α,β-diol ou α,ω-dicarboxylique, et notamment les polydiméthysiloxanes α,ω-diol et les polydiméthylsiloxanes α,ω-dicarboxylique; les polyarylsiloxanes α,ω-diol ou α,ω-dicarboxylique et notamment les polyphénylsiloxanes α,ω-diol ou α,ω-dicarboxylique; les polyarylsiloxanes à fonctions silanol tels que le polyphénylsiloxane; les polyalkylsiloxanes à fonctions silanol tels que le polydiméthylsiloxane; les polyaryl/alkylsiloxanes à fonctions silanol tels que le polyphényl/méthylsiloxane ou encore le polyphényl/propylsiloxane.
Tout particulièrement, on utilisera les polydiméthysiloxanes α,ω-diol de masse moléculaire moyenne en poids (Mw) comprise entre 400 et 10 000, voire entre 500 et 5000, et notamment entre 800 et 4000.
Lorsqu'elle est présente, ladite silicone peut de préférence représenter 0,1 à 15% en poids, notamment 1 à 10% en poids, voire 2 à 8% en poids, du poids du polycondensat.

Dans un mode de réalisation préféré de l'invention, l'acide monocarboxylique aromatique est présent en quantité molaire inférieure ou égale à celle de l'acide monocarboxylique non aromatique; notamment le rapport entre le nombre de mole d'acide monocarboxylique aromatique et le nombre de mole d'acide monocarboxylique non aromatique est de préférence compris entre 0,08 et 0,70, notamment entre 0,10 et 0,60, en particulier entre 0,12 et 0,40.
On a constaté que cela permet notamment d'obtenir un polymère avantageusement soluble dans les milieux huileux généralement employés pour formuler des compositions cosmétiques de type rouges à lèvres ou fonds de teint; par ailleurs, le film obtenu présente une rigidité et une souplesse adéquates pour son utilisation dans ce type de formulation, tout en ayant une brillance et une tenue de la brillance telles que recherchées.

De préférence, le polycondensat selon l'invention présente :
- un indice d'acide, exprimé en mg d'hydroxyde de potassium par g de polycondensat, supérieur ou égal à 1; notamment compris entre 2 et 30, et encore mieux compris entre 2,5 et 15; et/ou
- un indice d'hydroxyle exprimé en mg d'hydroxyde de potassium par g de polycondensat, supérieur ou égal à 40; notamment compris entre 40 et 120, et encore mieux compris entre 45 et 80.
Ces indices d'acide et d'hydroxyle peuvent être aisément déterminés par l'homme du métier par les méthodes analytiques habituelles.

De préférence, le polycondensat selon l'invention présente une masse moléculaire moyenne en poids (Mw) comprise entre 1500 et 300 000, voire entre 2000 et 200 000, et notamment entre 3000 et 100 000.

Le poids moléculaire moyen peut être déterminé par chromatographie par perméation sur gel ou par diffusion de la lumière, selon la solubilité du polymère considéré.

De préférence, le polycondensat selon l'invention présente une viscosité, mesurée à 110°C, comprise entre 20 et 4000 mPa.s, notamment entre 30 et 3500 mPa.s, voire entre 40 et 3000 mPa.s et encore mieux entre 50 et 2500 mPa.s. Cette viscosité est mesurée de la manière décrite avant les exemples.

Par ailleurs, le polycondensat est avantageusement soluble dans les milieux huileux cosmétiques usuellement employés, et notamment dans les huiles végétales, les alcanes, les esters gras, les alcools gras, les huiles de silicone, et plus particulièrement dans les milieux comprenant l'isododécane, le Parléam, l'isononanoate d'isononyle, l'octyldodécanol, la phényl triméthicone, le benzoate d'alkyle en C12-C15 et/ou la D5 (décaméthylcyclopentasiloxane).

Par soluble, on entend que le polymère forme une solution limpide dans au moins un solvant choisi parmi l'isododécane, le Parléam, l'isononanoate d'isononyle, l'octyldodécanol et le benzoate d'alkyle en C12-C15, à raison d'au moins 50% en poids, à 70°C. Certains composés présentent même une solubilité particulièrement avantageuse dans certains domaines d'applications, à savoir une solubilité dans au moins un des solvants cités ci-dessus, à raison d'au moins 50% en poids, à 25°C.

Le polycondensat selon l'invention peut être préparé par les procédés d'estérification/polycondensation usuellement employés par l'homme du métier. A titre d'illustration, un procédé général de préparation consiste :
- à mélanger le polyol et les acides monocarboxyliques aromatiques et non aromatiques,
- à chauffer le mélange sous atmosphère inerte, d'abord jusqu'à la température de fusion (généralement 100-130°C) et ensuite à une température comprise entre 150 et 220°C jusqu'à consommation complète des acides monocarboxyliques (atteint lorsque l'indice d'acide est inférieur ou égal à 1), de préférence en distillant au fur et à mesure l'eau formée, puis
- à éventuellement refroidir le mélange à une température comprise entre 90 et 150°C,
- à ajouter l'acide polycarboxylique et optionnellement la silicone à fonctions hydroxyles ou carboxyliques, en une seule fois ou de façon séquencée, puis
- à chauffer à nouveau à une température inférieure ou égale à 220°C, notamment comprise entre 170 et 220°C, de préférence en continuant à éliminer l'eau formée, jusqu'à l'obtention des caractéristiques requises en terme d'indice d'acide, de viscosité, d'indice d'hydroxyle et de solubilité.

Il est possible d'ajouter des catalyseurs d'estérification conventionnels, par exemple de type acide sulfonique (notamment à une concentration pondérale comprise entre 1 et 10%) ou type titanate (notamment à une concentration pondérale comprise entre 5 et 100 ppm).
Il est également possible de réaliser la réaction, en tout ou en partie, dans un solvant inerte tel que le xylène et/ou sous une pression réduite, pour faciliter l'élimination de l'eau.
Avantageusement, on n'utilise ni catalyseur ni solvant.

Ledit procédé de préparation peut comprendre en outre une étape d'addition d'au moins un agent antioxydant dans le milieu réactionnel, notamment à une concentration pondérale comprise entre 0,01 et 1%, par rapport au poids total de monomères, de façon à limiter les éventuelles dégradations liées à un chauffage prolongé.
L'agent antioxydant peut être de type primaire ou de type secondaire, et peut être choisi parmi les phénols encombrés, les amines secondaires aromatiques, les composés organophosphorés, les composés soufrés, les lactones, les bisphénols acrylés; et leurs mélanges.
Parmi les antioxydants particulièrement préférés, on peut notamment citer le BHT, le BHA , le TBHQ, le 1,3,5-trimethyl-2,4,6,tris(3,5-di-tertbutyl-4-hydroxybenzyl)-benzène, l'octadecyl-3,5,di-tertbutyl-4-hydroxycinnamate, le tetrakis-methylene-3-(3,5-di-tertbutyl-4-hydroxy-phenyl)propionate méthane, l'octadecyl-3-(3,5-di-tertbutyl-4-hydroxyphenyl)propionate 2,5-di-tertbutyl hydroquinone, le 2,2-methyl-bis-(4-methyl-6-tertbutyl phénol), le 2,2-methylene-bis-(4-ethyl-6-tertbutyl phénol), le 4,4-butylidene-bis(6-tertbutyl-m-cresol), le N,N-hexamethylene bis(3,5-di-tertbutyl-4-hydroxyhydrocinnamamide), le pentaerythritol tetrakis (3-(3,5-di-tertbutyl-4-hydroxyphenyl)propionate) notamment celui commercialisé par CIBA sous le nom IRGANOX 1010; l'octadecyl 3-(3,5-di-tertbutyl-4-hydroxphenyl) propionate notamment celui commercialisé par CIBA sous le nom IRGANOX 1076; la 1,3,5-tris(3,5-di-tertbutyl-4-hydroxybenzyl)-1,3,5-triazine-2,4,6(1H,3H,5H)trione notamment celui commercialisée par Mayzo of Norcross, Ga sous le nom BNX 3114; le di(stearyl)pentaerythritol diphosphite, le tris(2,4-ditertbutyl phenyl)phosphite notamment celui commercialisé par CIBA sous le nom IRGAFOS 168; le dilauryl thiodipropionate notamment celui commercialisé par CIBA sous le nom IRGANOX PS800; le bis(2,4-di-tertbutyl)pentaerythritol diphosphite notamment celui commercialisé par CIBA sous le nom IRGAFOS 126; le bis(2,4-bis)[2-phénylpropan-2-yl]phényl)pentaérythritol diphosphite, le triphénylphosphite, le (2,4-di-tertbutylphenyl)pentaerythritol diphosphite notamment celui commercialisé par GE Specialty Chemicals sous le nom ULTRANOX 626; le tris(nonylphenyl)phosphite notamment celui commercialisé par CIBA sous le nom IRGAFOS TNPP; le mélange 1:1 de N,N-hexamethylenebis(3,5-di-tertbutyl-4-hydroxy-hydrocinnamamide) et de tris(2,4-di-tertbutylphenyl)phosphate notamment celui commercialisé par CIBA sous le nom Irganox B 1171; le tétrakis (2,4-di-tert-butylphényl)phosphite notamment celui commercialisé par CIBA sous le nom IRGAFOS P-EPQ; le distéarylthiodipropionate notamment celui commercialisé par CIBA sous le nom IRGANOX PS802; le 2,4-bis(octylthiométhyl)o-crésol notamment celui commercialisé par CIBA sous le nom IRGANOX 1520; le 4,6-bis(dodécylthiométhyl)o-crésol notamment celui commercialisé par CIBA sous le nom IRGANOX 1726.

Les polycondensats selon l'invention peuvent être utilisés très avantageusement dans une composition notamment cosmétique ou pharmaceutique, qui comprend par ailleurs un milieu physiologiquement, notamment cosmétiquement ou pharmaceutiquement, acceptable, c'est-à-dire un milieu compatible avec les tissus cutanés comme la peau du visage ou du corps, et les matières kératiniques telles que les cheveux, les cils, les sourcils et les ongles.
La quantité de polycondensat présente dans les compositions dépend bien entendu du type de composition et des propriétés recherchées et peut varier à l'intérieur d'une gamme très large, comprise généralement entre 0,1 et 70% en poids, de préférence entre 1 et 50% en poids, notamment entre 10 et 45% en poids, voire entre 20 et 40% en poids, et mieux entre 25 et 35% en poids, par rapport au poids de la composition cosmétique ou pharmaceutique finale.

La composition peut alors comprendre, selon l'application envisagée, les constituants habituels à ce type de composition.
La composition selon l'invention peut avantageusement comprendre une phase grasse liquide, qui peut constituer un milieu solvant des polymères selon l'invention, et qui peut comprendre au moins un composé choisi parmi les huiles et/ou solvants d'origine minérale, animale, végétale ou synthétique, carbonés, hydrocarbonés, fluorés et/ou siliconés, volatils ou non volatils, seuls ou en mélange dans la mesure où ils forment un mélange homogène et stable et sont compatibles avec l'utilisation envisagée.

Par 'volatil', on entend au sens de l'invention, tout composé susceptible de s'évaporer au contact des matières kératiniques, ou des lèvres, en moins d'une heure, à température ambiante (25°C) et pression atmosphérique (1 atm). Notamment, ce composé volatil a une pression de vapeur non nulle, à température ambiante et pression atmosphérique, notamment allant de 0,13 Pa à 40 000 Pa (10⁻³ à 300 mm de Hg), en particulier allant de 1,3 Pa à 13 000 Pa (0,01 à 100 mm de Hg), et plus particulièrement allant de 1,3 Pa à 1300 Pa (0,01 à 10 mm de Hg).
Par opposition, on entend par 'non volatil', un composé restant sur les matières kératiniques ou les lèvres à température ambiante et pression atmosphérique, au moins une heure et ayant notamment une pression de vapeur inférieure à 10⁻³ mm de Hg (0,13Pa).

De préférence, le milieu physiologiquement acceptable de la composition selon l'invention peut comprendre, dans une phase grasse liquide, au moins une huile et/ou un solvant qui peut être choisi parmi, seul ou en mélange :
1/ les esters des acides monocarboxyliques avec les monoalcools et polyalcools; avantageusement, ledit ester est un benzoate d'alkyle en C12-C15 ou répond à la formule suivante : R'₁-COO-R'₂ où :
   R'₁ représente un radical alkyle linéaire ou ramifié de 1 à 40 atomes de carbone, de préférence de 7 à 19 atomes de carbone, comprenant éventuellement une ou plusieurs double liaisons éthyléniques, éventuellement substitué et dont la chaîne hydrocarbonée peut être interrompue par un ou plusieurs hétéroatomes choisis parmi N et O et/ou une ou plusieurs fonctions carbonyles, et
   R'₂ représente un radical alkyle linéaire ou ramifié de 1 à 40 atomes de carbone, de préférence de 3 à 30 atomes de carbone et mieux de 3 à 20 atomes de carbone, comprenant éventuellement une ou plusieurs double liaisons éthyléniques, éventuellement substitué et dont la chaîne hydrocarbonée peut être interrompue par un ou plusieurs hétéroatomes choisis parmi N et O et/ou une ou plusieurs fonctions carbonyles.
   Par "éventuellement substitué", on entend que R'₁ et/ou R'₂ peuvent porter un ou plusieurs substituants choisis, par exemple, parmi les groupements comprenant un ou plusieurs hétéroatomes choisi parmi O et/ou N, tels que amino, amine, alcoxy, hydroxyle.
   Des exemples des groupes R'₁ sont ceux dérivés des acides gras de préférence supérieur choisis dans le groupe constitué des acides acétique, propionique, butyrique, caproïque, caprylique, pélargonique, caprique, undécanoïque, laurique, myristique, palmitique, stéarique, isostéarique, arachidique, béhénique, oléique, linolénique, linoléïque, oléostéarique, arachidonique, érucique, et de leurs mélanges.
   De préférence, R'₁ est un groupe alkyle ramifié non substitué de 4 à 14 atomes de carbone, de préférence de 8 à 10 atomes de carbone et R₂ est un groupe alkyle ramifié non substitué de 5 à 15 atomes de carbone, de préférence de 9 à 11 atomes de carbone.
   On peut en particulier citer, de préférence, les esters en C₈-C₄₈, éventuellement incorporant dans leur chaîne hydrocarbonée un ou plusieurs hétéroatomes parmi N et O et/ou une ou plusieurs fonctions carbonyle; et plus particulièrement l'huile de purcellin (octanoate de cétostéaryle), l'isononanoate d'isononyle, le myristate d'isopropyle, le palmitate d'isopropyle, le palmitate d'éthyl-2-hexyle, le stéarate d'octyl-2-dodécyle, l'érucate d'octyl-2-dodécyle, l'isostéarate d'isostéaryle, le benzoate d'alcool en C₁₂ à C₁₅, le laurate d'hexyle, l'adipate de diisopropyle; et les heptanoates, octanoates, décanoates ou ricinoléates d'alcools ou de polyalcools, par exemple d'alcools gras comme le dioctanoate de propylène glycol, ainsi que le N-lauroyl sarcosinate d'isopropyle (notamment Eldew-205SL d'Ajinomoto); les esters hydroxylés comme le lactate d'isostéaryle, le malate de di-isostéaryle; et les esters du pentaérythritol; les esters ramifiés en C8-C16, notamment le néopentanoate d'isohexyle.
2/ les huiles végétales hydrocarbonées à forte teneur en triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées; ces huiles sont notamment les huiles de germe de blé, de maïs, de tournesol, de karité, de ricin, d'amandes douces, de macadamia, d'abricot, de soja, de colza, de coton, de luzerne, de pavot, de potimarron, de sésame, de courge, d'avocat, de noisette, de pépins de raisin ou de cassis, d'onagre, de millet, d'orge, de quinoa, d'olive, de seigle, de carthame, de bancoulier, de passiflore, de rosier muscat, de jojoba, de palme, de calophyllum; ou encore les triglycérides des acides caprylique/caprique comme ceux vendus par la société Stearinerie Dubois ou ceux vendus sous les dénominations "Miglyol 810^{®}", "812^{®} " et "818^{®} " par la société Dynamit Nobel.
3/ les alcools, et notamment les monoalcools, en C6-C32, notamment C12-C26, comme l'alcool oléïque, l'alcool linoléique, l'alcool linolénique, l'alcool isostéarylique, le 2-hexyldécanol, le 2-butyloctanol, le 2-undécylpentadécanol et l'octyldodécanol;
4/ les huiles hydrocarbonées, linéaires ou ramifiés, volatiles ou non, d'origine synthétique ou minérale, qui peuvent être choisies parmi les huiles hydrocarbonées ayant de 5 à 100 atomes de carbones, et notamment la vaseline, les polydécènes, les polyisobutènes hydrogénés tel que le Parléam, le squalane, le perhydrosqualène et leurs mélanges.
   On peut plus particulièrement citer les alcanes linéaires, ramifiés et/ou cycliques en C5-C48, et préférentiellement les alcanes ramifiés en C8-C16 comme les isoalcanes en C8-C16 d'origine pétrolière (appelées aussi isoparaffines); notamment le décane, l'heptane, le dodécane, le cyclohexane; ainsi que l'isododécane, l'isodécane, l'isohexadécane.
5/ les huiles de silicone, volatiles ou non volatiles;
   Comme huiles de silicone volatiles, on peut citer les huiles de silicones linéaires
   ou cycliques volatiles, notamment celles ayant une viscosité inférieure à 8 centistokes, et ayant notamment de 2 à 10 atomes de silicium, ces silicones comportant éventuellement des groupes alkyles ou alkoxy ayant de 1 à 22 atomes de carbone; et en particulier l'octaméthylcyclotétrasiloxane, le décaméthylcyclopentasiloxane, le dodécaméthylcyclohexasiloxane, l'heptaméthylhexyltrisiloxane, l'heptaméthyloctyltrisiloxane, l'hexaméthyldisiloxane, l'octaméthyltrisiloxane, le décaméthyltétrasiloxane, le dodécaméthylpentasiloxane, le méthylhexyldiméthylsiloxane et leurs mélanges.
   Les huiles de silicone non volatiles utilisables selon l'invention peuvent être les polydiméthylsiloxanes (PDMS), les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényltriméthicones, les phényldiméthicones, les phényltriméthylsiloxydiphénylsiloxanes, les diphényldiméthicones, les diphénylméthyldiphényltrisiloxanes, les 2-phényléthyl triméthylsiloxysilicates.

Préférentiellement, le milieu physiologiquement acceptable de la composition selon l'invention comprend, dans une phase grasse liquide, au moins une huile et/ou un solvant choisi parmi, seul ou en mélange, l'isododécane, le Parléam, l'isononanoate d'isononyle, l'octyldodécanol, la phényl triméthicone, les benzoates d'alkyle en C12-C15 et/ou la D5 (décaméthylcyclopentasiloxane).

La phase grasse liquide peut en outre comprendre des huiles et/ou solvants additionnels, qui peuvent être choisis parmi, seul ou en mélange :
- les huiles fluorées telles que les perfluoropolyéthers, les perfluoroalcanes comme la perfluorodécaline, les perfluorodamantanes, les monoesters, diesters et triesters de perfluoroalkylphosphates et les huiles esters fluorés;
- les huiles d'origine animale;
- les éthers en C₆ à C_{40,} notamment en C10-C40; les éthers de propylène glycol liquides à température ambiante tels que le monométhyléther de propylène glycol, l'acétate de monométhyléther de propylène glycol, le mono n-butyl éther de dipropylène glycol ;
- les acides gras en C₈-C₃₂, comme l'acide oléique, l'acide linoléique, l'acide linolénique et leurs mélanges.
- les huiles bifonctionnelles, comprenant deux fonctions choisies parmi ester et/ou amide et comprenant de 6 à 30 atomes de carbone, notamment 8 à 28 atomes de carbone, mieux de 10 à 24 carbones, et 4 hétéroatomes choisis parmi O et N; de préférence les fonctions amide et ester étant dans la chaîne;
- les cétones liquides à température ambiante (25°C) tels que la méthyléthylcétone, la méthylisobutylcétone, la diisobutylcétone, l'isophorone, la cyclohexanone, l'acétone ;
- les aldéhydes liquides à température ambiante tels que le benzaldéhyde, l'acétaldéhyde;

La phase grasse liquide peut représenter 1 à 90% en poids de la composition, notamment de 5 à 75% en poids, en particulier de 10 à 60% en poids, voire de 25 à 55% en poids, du poids total de la composition.

La composition selon l'invention peut comprendre avantageusement un agent épaississant qui peut en particulier être choisi parmi :
- les silices notamment hydrophobes, telles que celles décrites dans le document EP-A-898960, et par exemple commercialisées sous les références "AEROSIL R812^{®}" par la société Degussa, "CAB-O-SIL TS-530^{®}", "CAB-O-SIL TS-610^{®}", "CAB-O-SIL TS-720^{®}"par la société Cabot, "AEROSIL R972^{®}", "AEROSIL R974^{®}" par la société Degussa ;
- les argiles telles que la montmorillonite, les argiles modifiées telles que les bentones par exemple, l'hectorite stéaralkonium, la bentonite stéaralkonium,
- les alkyléther de polysaccharides (notamment dont le groupe alkyle comporte de 1 à 24 atomes de carbones, de préférence de 1 à 10, mieux de 1 à 6, et plus spécialement de 1 à 3) tels que ceux décrits dans le document EP-A-898958.
La quantité d'agent épaississant dans la composition selon l'invention peut aller de 0,05 à 40% en poids, par rapport au poids total de la composition, de préférence de 0,5 à 20% et mieux de 1 à 15% en poids.

La composition selon l'invention peut également comprendre au moins une cire d'origine végétale, animale, minérale ou de synthèse, voire siliconée.
On peut en particulier citer, seules ou en mélange, les cires hydrocarbonées telles que la cire d'abeilles; la cire de Carnauba, de Candellila, d'Ouricoury, du Japon, les cires de fibres de lièges ou de canne à sucre; les cires de paraffine, de lignite; les cires microcristallines; la cire de lanoline; la cire de Montan; les ozokérites; les cires de polyéthylène; les cires obtenues par synthèse de Fischer-Tropsch; les huiles hydrogénées, les esters gras et les glycérides concrets à 25°C. On peut également utiliser des cires de silicone, parmi lesquelles on peut citer les alkyls, alcoxys et/ou esters de polyméthylsiloxane.
La quantité de cire dans la composition selon l'invention peut aller de 0,1 à 70% en poids, par rapport au poids total de la composition, de préférence de 1 à 40% en poids, et mieux de 5 à 30% en poids.

La composition selon l'invention peut également comprendre une ou plusieurs matières colorantes choisies parmi les composés pulvérulents comme les pigments, les charges, les nacres et les paillettes, et/ou les colorants liposolubles ou hydrosolubles.
Les matières colorantes, notamment pulvérulentes, peuvent être présentes, dans la composition, en une teneur de 0,01 à 50% en poids, par rapport au poids de la composition, de préférence de 0,1 à 40% en poids, voire de 1 à 30% en poids.
Par pigments, il faut comprendre des particules de toute forme, blanches ou colorées, minérales ou organiques, insolubles dans le milieu physiologique, destinées à colorer la composition.
Par nacres, il faut comprendre des particules de toute forme irisées, notamment produites par certains mollusques dans leur coquille ou bien synthétisées.
Les pigments peuvent être blancs ou colorés, minéraux et/ou organiques, interférentiels ou non. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.
Les charges peuvent être minérales ou organiques, lamellaires ou sphériques. On peut citer le talc, le mica, la silice, le kaolin, les poudres de Nylon et de polyéthylène, de poly-β-alanine et de polyéthylène, le Téflon, la lauroyl-lysine, l'amidon, le nitrure de bore, les poudres de polymères de tétrafluoroéthylène, les microsphères creuses telles que l'Expancel (Nobel Industrie), le polytrap (Dow Corning) et les microbilles de résine de silicone (Tospearls de Toshiba, par exemple), le carbonate de calcium précipité, le carbonate et l'hydro-carbonate de magnésium, l'hydroxyapatite, les microsphères de silice creuses (SILICA BEADS de MAPRECOS), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.
Les colorants liposolubles sont par exemple le rouge Soudan, le DC Red 17, le DC Green 6, le β-carotène, l'huile de soja, le brun Soudan, le DC Yellow, 11, le DC Violet 2, le DC orange 5, le jaune quinoléine. Ils peuvent représenter 0,01 à 20% du poids de la composition et mieux de 0,1 à 6 %.
Les colorants hydrosolubles sont par exemple le jus de betterave, le bleu de méthylène et peuvent représenter 0,01 à 6% du poids total de la composition.

La composition peut comprendre, en outre, d'autres ingrédients utilisés couramment dans les compositions cosmétiques. De tels ingrédients peuvent être choisis parmi les antioxydants, les parfums, les huiles essentielles, les conservateurs, les actifs cosmétiques, les hydratants, les vitamines, les céramides, les filtres solaires, les tensioactifs, les agents d'étalement, les agents mouillants, les agents dispersants, les anti-mousses, les neutralisants, les stabilisants, les polymères et notamment les polymères filmogènes liposolubles, et leurs mélanges.
Bien entendu, l'homme du métier veillera à choisir ce ou ces éventuels composés complémentaires, et/ou leur quantité, de manière telle que les propriétés avantageuses de la composition pour l'utilisation selon l'invention ne soient pas, ou substantiellement pas, altérées par l'adjonction envisagée.

Les compositions selon l'invention peuvent se présenter sous toute forme acceptable et usuelle pour une composition cosmétique ou pharmaceutique.
Elles peuvent donc se présenter sous la forme d'une suspension, une dispersion notamment d'huile dans de l'eau grâce à des vésicules; une solution organique ou huileuse éventuellement épaissie voire gélifiée; une émulsion huile-dans-eau, eau-dans-huile, ou multiple; un gel ou une mousse; un gel huileux ou émulsionné; une dispersion de vésicules notamment lipidiques; une lotion biphase ou multiphase; un spray; d'une lotion, d'une crème, d'une pommade, d'une pâte souple, d'un onguent, d'un solide coulé ou moulé et notamment en stick ou en coupelle, ou encore de solide compacté.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

Les compositions conformes à l'invention présentant une brillance et une tenue de ladite brillance améliorées par rapport à l'état de l'art, elles peuvent être utilisées pour le soin ou le maquillage des matières kératiniques telles que les cheveux, la peau, les cils, les sourcils, les ongles, les lèvres, le cuir chevelu et plus particulièrement pour le maquillage des lèvres, des cils et/ou du visage.
Elles peuvent donc se présenter sous la forme d'un produit de soin et/ou de maquillage de la peau du corps ou du visage, des lèvres, des cils, des sourcils, des cheveux, du cuir cheveu ou des ongles; d'un produit solaire ou autobronzant; d'un produit capillaire notamment de coloration, de conditionnement et/ou de soin des cheveux; elles se présentent avantageusement sous forme de mascara, de rouge à lèvres, de brillant à lèvres (gloss), de fard à joues ou à paupières, de fond de teint.

L'invention a encore pour objet un procédé de traitement cosmétique des matières kératiniques, notamment de la peau du corps ou du visage, des lèvres, des ongles, des cheveux et/ou des cils, comprenant l'application sur lesdites matières d'une composition cosmétique telle que définie précédemment.
Ce procédé selon l'invention permet notamment le soin ou le maquillage des lèvres, par application d'une composition de rouge à lèvres ou de brillant à lèvres (gloss) selon l'invention.

L'invention est illustrée plus en détail dans les exemples suivants.

### Méthode de mesure de la viscosité

La viscosité à 80°C ou à 110°C du polymère est mesurée à l'aide d'un viscosimètre à cône plan de type BROOKFIELD CAP 1000+.
Le cône-plan adapté est déterminé par l'homme du métier, sur la base de ses connaissances; notamment :
- entre 50 et 500 mPa.s, on peut utiliser un cône 02
- entre 500 et 1000 mPa.s : cône 03
- entre 1000 et 4000 mPa.s : cône 05
- entre 4000 et 10000 mPa.s : cône 06

### Exemple 1 : Synthèse du pentaérythrityl benzoate/isophtalate/isostéarate/ PDMS

Dans un réacteur équipé d'une agitation mécanique, d'une arrivée d'argon et d'un système de distillation, on charge 32 g d'acide benzoïque, 504 g d'acide isostéarique et 136 g de pentaérythritol, puis on chauffe progressivement, sous un léger courant d'argon, à 110-130°C pour obtenir une solution homogène. On augmente ensuite progressivement la température jusqu'à 180°C et on la maintient pendant environ 2 heures. On augmente de nouveau la température jusqu'à 220°C et on la maintient jusqu'à ce qu'on obtienne un indice d'acide inférieur ou égal à 1, ce qui prend environ 9 heures. On refroidit à une température comprise entre 100 et 130°C puis on introduit 144 g d'acide isophtalique et 80 g de Silicone α,ω diol X22-160AS de Shin-Etsu, et on chauffe à nouveau progressivement jusqu'à 220°C pendant environ 15 heures.
On obtient ainsi 750 g de polycondensat pentaérythrityl benzoate/isophtalate/isostéarate /PDMS sous la forme d'une huile.

Le polycondensat présente les caractéristiques suivantes :
- soluble à 50% en poids, à 25°C, dans le Parléam
- Indice d'acide = 12,8
- Indice d'hydroxyle = 42
- Mw = 11700
- η_{80°C} = 798 mPa.s
- η_{110°C} = 190 mPa.s
- rapport entre le nombre de mole d'acide monocarboxylique aromatique et le nombre de mole d'acide monocarboxylique non aromatique : 0,15.

### Exemple 2 : Synthèse du pentaérythrityl benzoate/isophtalate/isostéarate

Dans un réacteur équipé d'une agitation mécanique, d'une arrivée d'argon et d'un système de distillation, on charge 20 g d'acide benzoïque, 280 g d'acide isostéarique et 100 g de pentaérythritol, puis on chauffe progressivement, sous un léger courant d'argon, à 110-130°C pour obtenir une solution homogène. On augmente ensuite progressivement la température jusqu'à 180°C et on la maintient pendant environ 2 heures. On augmente de nouveau la température jusqu'à 220°C et on la maintient jusqu'à ce qu'on obtienne un indice d'acide inférieur ou égal à 1, ce qui prend environ 11 heures. On refroidit à une température comprise entre 100 et 130°C puis on introduit 100 g d'acide isophtalique et on chauffe à nouveau progressivement jusqu'à 220°C pendant environ 11 heures.
On obtient ainsi 405 g de polycondensat pentaérythrityl benzoate/isophtalate/isostéarate sous la forme d'une huile très épaisse.

Le polycondensat présente les caractéristiques suivantes :
- soluble à 50% en poids, à 25°C, dans le Parléam
- Indice d'acide = 3,7
- Indice d'hydroxyle = 72
- Mw = 59400
- η_{110°C} = 1510 mPa.s
- rapport entre le nombre de mole d'acide monocarboxylique aromatique et le nombre de mole d'acide monocarboxylique non aromatique : 0,16.

### Exemple 3 : Synthèse du pentaérythrityl benzoate/isophtalate/isostéarate

Dans un réacteur équipé d'une agitation mécanique, d'une arrivée d'argon et d'un système de distillation, on charge 35 g d'acide benzoïque, 270 g d'acide isostéarique et 80 g de pentaérythritol puis on chauffe progressivement, sous un léger courant d'argon, à 110-130°C pour obtenir une solution homogène. On augmente ensuite progressivement la température jusqu'à 180°C et on la maintient pendant environ 2 heures. On augmente de nouveau la température jusqu'à 220°C et on la maintient jusqu'à ce qu'on obtienne un indice d'acide inférieur ou égal à 1, ce qui prend environ 11 heures. On refroidit à une température comprise entre 100 et 130°C puis on introduit 65 g d'acide isophtalique et on chauffe à nouveau progressivement jusqu'à 220°C pendant environ 5 heures.
On obtient ainsi 380 g de polycondensat pentaérythrityl benzoate/isophtalate/isostéarate sous la forme d'une huile.

Le polycondensat présente les caractéristiques suivantes :
- soluble à 50% en poids, à 25°C, dans le Parléam
- Indice d'acide = 5,5
- Indice d'hydroxyle = 103
- Mw = 7200
- η_{80°C} = 700 mPa.s
- rapport entre le nombre de mole d'acide monocarboxylique aromatique et le nombre de mole d'acide monocarboxylique non aromatique : 0,30.

### Exemple 4 : Synthèse du pentaérythrityl benzoate/isophtalate/isostéarate/ PDMS

Dans un réacteur équipé d'une agitation mécanique, d'une arrivée d'argon et d'un système de distillation, on charge 14 g d'acide benzoïque, 255 g d'acide isostéarique et 75 g de pentaérythritol, puis on chauffe progressivement, sous un léger courant d'argon, à 110-130°C pour obtenir une solution homogène. On augmente ensuite progressivement la température jusqu'à 180°C et on la maintient pendant environ 2 heures. On augmente de nouveau la température jusqu'à 220°C et on la maintient jusqu'à ce qu'on obtienne un indice d'acide inférieur ou égal à 1, ce qui prend environ 7 heures. On refroidit à une température comprise entre 100 et 130°C puis on introduit 65 g d'acide isophtalique et 38 g de Silicone α,ω dicarboxy Tegomer C-Si 2342 (Goldschmidt), et on chauffe à nouveau progressivement jusqu'à 220°C pendant environ 15 heures.
On obtient ainsi 375 g de polycondensat pentaérythrityl benzoate/isophtalate/isostéarate /PDMS sous la forme d'une huile épaisse.

Le polycondensat présente les caractéristiques suivantes :
- soluble à 50% en poids, à 25°C, dans le Parléam
- Indice d'acide = 5,5
- Indice d'hydroxyle = 110
- η_{80°C} = 1332 mPa.s
- rapport entre le nombre de mole d'acide monocarboxylique aromatique et le nombre de mole d'acide monocarboxylique non aromatique : 0,12.

### Exemple 5 : Synthèse du pentaérythrityl benzoate/isophtalate/isostéarate/ PDMS

Dans un réacteur équipé d'une agitation mécanique, d'une arrivée d'argon et d'un système de distillation, on charge 16 g d'acide benzoïque, 252 g d'acide isostéarique et 68 g de pentaérythritol, puis on chauffe progressivement, sous un léger courant d'argon, à 110-130°C pour obtenir une solution homogène. On augmente ensuite progressivement la température jusqu'à 180°C et on la maintient pendant environ 2 heures. On augmente de nouveau la température jusqu'à 220°C et on la maintient jusqu'à ce qu'on obtienne un indice d'acide inférieur ou égal à 1, ce qui prend environ 9 heures. On refroidit à une température comprise entre 100 et 130°C puis on introduit 40 g d'acide isophtalique et 40 g de Silicone α,ω diol X22-160AS de Shin-Etsu, et on chauffe à nouveau progressivement jusqu'à 220°C pendant environ 7 heures.
On obtient ainsi 345 g de polycondensat pentaérythrityl benzoate/isophtalate/isostéarate /PDMS sous la forme d'une huile.

Le polycondensat présente les caractéristiques suivantes :
- soluble à 50% en poids, à 25°C, dans le Parléam
- Indice d'acide = 2,5
- Indice d'hydroxyle = 63
- Mw = 3600
- η_{80°C} = 125 mPa.s
- rapport entre le nombre de mole d'acide monocarboxylique aromatique et le nombre de mole d'acide monocarboxylique non aromatique : 0,15.

### Exemple 6 : Synthèse du pentaérythrityl benzoate/isophtalate/stéarate

Dans un réacteur équipé d'une agitation mécanique, d'une arrivée d'argon et d'un système de distillation, on charge 10 g d'acide benzoïque, 370 g d'acide stéarique et 95 g de pentaérythritol puis on chauffe progressivement, sous un léger courant d'argon, à 110-130°C pour obtenir une solution homogène. On augmente ensuite progressivement la température jusqu'à 180°C et on la maintient pendant environ 2 heures. On augmente de nouveau la température jusqu'à 220°C et on la maintient jusqu'à ce qu'on obtienne un indice d'acide inférieur ou égal à 1, ce qui prend environ 11 heures. On refroidit à une température comprise entre 100 et 130°C puis on introduit 90 g d'acide isophtalique et on chauffe à nouveau progressivement jusqu'à 220°C pendant environ 11 heures.
On obtient ainsi 430 g de polycondensat pentaérythrityl benzoate/isophtalate/stéarate sous la forme d'une huile très épaisse.

Le polycondensat présente les caractéristiques suivantes :
- soluble à 50% en poids, à 70°C, dans le Parléam
- Indice d'acide = 10,8
- Mw = 8800
- η_{80°C} = 360 mPa.s

### Exemple 7

De manière similaire aux exemples précédents, on prépare le polycondensat suivant (les % sont en poids):

| | Polyol (% et nature) | Acide aromatique (% et nature) | Acide polycarboxylique ou anhydride (% et nature) | Acide non aromatique (% et nature) | Solubilité * |
|---|---|---|---|---|---|
| Exemple A | 21,6 Pentaérythritol | 3,9 benzoïque | 19,5 Acide isophtalique | 27,5% isostéarique + 27,5% isononanoïque | à 25°C |

| | | | | | |
|---|---|---|---|---|---|
| * 'à 25°C' indique que le polymère est soluble à 50% en poids, à 25°C, dans le Parléam; 'à 70°C' indique que le polymère est soluble à 50% en poids, à 70°C, dans le Parléam | | | | | |

### Exemple 8

On a préparé un gloss ayant la composition suivante :

| | |
|---|---|
| - polycondensat de l'exemple 1 | 29 g |
| - polybutène | 34 g |
| - isononanoate d'isononyle | 4 g |
| - octyldodécanol | 10 g |
| - silice (Aérosil R972) | 5 g |
| - trimellitate de tridécyle | qsp 100g |

On obtient, après application sur les lèvres, un film brillant et qui le reste au moins 2 heures.

### Exemple 9

On a préparé un gloss ayant la composition suivante :

| | |
|---|---|
| - polycondensat de l'exemple 2 | 28 g |
| - polybutène | 34 g |
| - isononate d'isononyle | 4 g |
| - octyldodécanol | 10 g |
| - silice (Aérosil R972) | 5 g |
| - trimellitate de tridécyle | qsp 100g |

On obtient, après application sur les lèvres, un film brillant et qui le reste au moins 2 heures.

### Exemple 10

On a préparé un rouge à lèvres ayant la composition suivante :

| | |
|---|---|
| - polycondensat de l'exemple 2 | 30 g |
| - cire de polyéthylène | 11 g |
| - pigments et charges | 7 g |
| - Parléam (isoparaffine hydrogénée) | qsp 100 g |

On obtient, après application sur les lèvres, un film coloré et brillant, qui reste brillant au moins 2 heures.

### Exemple 11

On a préparé un rouge à lèvres ayant la composition suivante :

| | |
|---|---|
| - polycondensat de l'exemple 6 | 10 g |
| - cire de polyéthylène | 11 g |
| - pigments et charges | 7 g |
| - Parléam (isoparaffine hydrogénée) | qsp 100 g |

## Revendications

1. Composition cosmétique ou pharmaceutique comprenant, dans un milieu cosmétiquement ou pharmaceutiquement acceptable, au moins un polycondensat susceptible d'être obtenu par réaction :
- de 10 à 30% en poids, par rapport au poids total du polycondensat, de pentaérytritol ;
- de 30 à 80% en poids, par rapport au poids total du polycondensat, d'au moins un acide monocarboxylique non aromatique, saturé ou insaturé, linéaire, ramifié et/ou cyclique, comprenant 6 à 32 atomes de carbone choisi parmi l'acide isostéarique seul ou l'acide stéarique seul ;
- de 0,1 à 10 % en poids, par rapport au poids total du polycondensat, d'acide benzoïque ;
- de 5 à 40% en poids, par rapport au poids total du polycondensat, d'acide isophtalique.

2. Composition selon la revendications précédentes, dans laquelle le pentaérythritol représente 12 à 25% en poids, et mieux 14 à 22% en poids, du poids total du polycondensat.

3. Composition selon l'une des revendications précédentes, dans laquelle l'acide isostéarique seul ou l'acide stéarique seul représente 40 à 75% en poids, voire 45 à 70% en poids, et mieux 50 à 65% en poids, du poids total du polycondensat final.

4. Composition selon l'une des revendications précédentes, dans laquelle l'acide benzoïque représente 0,5 à 9,95% en poids, mieux de 1 à 9,5% en poids, voire 1,5 à 8% en poids, du poids total du polycondensat final.

5. Composition selon l'une des revendications précédentes, dans laquelle l'acide isophtalique représente 10 à 30% en poids, et mieux 14 à 25% en poids, du poids total du polycondensat.

6. Composition selon l'une des revendications précédentes, dans laquelle le rapport entre le nombre de mole d'acide benzoïque et le nombre de mole d'acide isostéarique seul ou d'acide stéarique seul est compris entre 0,08 et 0,70, en particulier entre 0,10 et 0,60, voire entre 0,12 et 0,40.

7. Composition selon l'une des revendications précédentes, dans laquelle le polycondensat présente au moins l'une des caractéristiques suivantes :
- un indice d'acide, exprimé en mg d'hydroxyde de potassium par g de polycondensat, supérieur ou égal à 1; notamment compris entre 2 et 30, et encore mieux compris entre 2,5 et 15; et/ou
- un indice d'hydroxyle exprimé en mg d'hydroxyde de potassium par g de polycondensat, supérieur ou égal à 40; notamment compris entre 40 et 120, et encore mieux compris entre 45 et 80; et/ou
- une masse moléculaire moyenne en poids (Mw) comprise entre 1500 et 300 000, voire entre 2000 et 200 000, et notamment entre 3000 et 100 000; et/ou
- une viscosité, mesurée à 110°C, comprise entre 20 et 4000 mPa.s, notamment entre 30 et 3500 mPa.s, voire entre 40 et 3000 mPa.s et encore mieux entre 50 et 2500 mPa.s; et/ou
- une solubilité dans au moins un solvant choisi parmi l'isododécane, le Parléam, l'isononanoate d'isononyle, l'octyldodécanol et le benzoate d'alkyle en C12-C15, à raison d'au moins 50% en poids, à 70°C.

8. Composition selon l'une des revendications précédentes, dans laquelle le polycondensat est présent en une quantité comprise entre 0,1 et 70% en poids, de préférence entre 1 et 50% en poids, notamment entre 10 et 45% en poids, voire entre 20 et 40% en poids, et mieux entre 25 et 35% en poids, par rapport au poids de la composition cosmétique ou pharmaceutique finale.

9. Composition selon l'une des revendications précédentes, dans laquelle le milieu cosmétiquement ou pharmaceutiquement acceptable comprend au moins une phase grasse liquide, qui peut comprendre au moins un composé choisi parmi les huiles et/ou solvants d'origine minérale, animale, végétale ou synthétique, carbonés, hydrocarbonés, fluorés et/ou siliconés, volatils ou non volatils, seuls ou en mélange.

10. Composition selon l'une des revendications précédentes, dans laquelle le milieu cosmétiquement ou pharmaceutiquement acceptable comprend au moins une huile et/ou un solvant choisi parmi, seul ou en mélange :
1/ les esters des acides monocarboxyliques avec les monoalcools et polyalcools; avantageusement, ledit ester est un benzoate d'alkyle en C12-C15 ou répond à la formule suivante : R'₁-COO-R'₂ où :
R'₁ représente un radical alkyle linéaire ou ramifié de 1 à 40 atomes de carbone, comprenant éventuellement une ou plusieurs double liaisons éthyléniques, éventuellement substitué et dont la chaîne hydrocarbonée peut être interrompue par un ou plusieurs hétéroatomes choisis parmi N et O et/ou une ou plusieurs fonctions carbonyles, et
R'₂ représente un radical alkyle linéaire ou ramifié de 1 à 40 atomes de carbone, comprenant éventuellement une ou plusieurs double liaisons éthyléniques, éventuellement substitué et dont la chaîne hydrocarbonée peut être interrompue par un ou plusieurs hétéroatomes choisis parmi N et O et/ou une ou plusieurs fonctions carbonyles.
2/ les huiles végétales hydrocarbonées à forte teneur en triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées;
3/ les alcools, et notamment les monoalcools, en C6-C32, notamment C12-C26, comme l'alcool oléïque, l'alcool linoléique, l'alcool linolénique, l'alcool isostéarylique, le 2-hexyldécanol, le 2-butyloctanol, le 2-undécylpentadécanol et l'octyldodécanol;
4/ les huiles hydrocarbonées, linéaires ou ramifiés, volatiles ou non, d'origine synthétique ou minérale, qui peuvent être choisies parmi les huiles hydrocarbonées ayant de 5 à 100 atomes de carbones, et notamment la vaseline, les polydécènes, les polyisobutènes hydrogénés tel que le Parléam, le squalane, le perhydrosqualène, les alcanes linéaires, ramifiés et/ou cycliques en C5-C48, et leurs mélanges.
5/ les huiles de silicone, volatiles ou non volatiles;

11. Composition selon l'une des revendications précédentes, dans laquelle le milieu cosmétiquement ou pharmaceutiquement acceptable comprend au moins une huile et/ou un solvant choisi parmi, seul ou en mélange, l'isododécane, le Parléam, l'isononanoate d'isononyle, l'octyldodécanol, la phényl triméthicone, les benzoates d'alkyle en C12-C15 et/ou la D5 (décaméthylcyclopentasiloxane).

12. Composition selon l'une des revendications précédentes, dans laquelle le milieu cosmétiquement ou pharmaceutiquement acceptable comprend au moins un composé choisi parmi les agents épaississants, les cires, les matières colorantes, les antioxydants, les parfums, les huiles essentielles, les conservateurs, les actifs cosmétiques, les hydratants, les vitamines, les céramides, les filtres solaires, les tensioactifs, les agents d'étalement, les agents mouillants, les agents dispersants, les anti-mousses, les neutralisants, les stabilisants, les polymères et notamment les polymères filmogènes liposolubles, et leurs mélanges.

13. Composition selon l'une des revendications précédentes, se présentant sous la forme d'un produit de soin et/ou de maquillage de la peau du corps ou du visage, des lèvres, des cils, des sourcils, des cheveux, du cuir cheveu ou des ongles; d'un produit solaire ou autobronzant; d'un produit capillaire notamment de coloration, de conditionnement et/ou de soin des cheveux.

14. Composition selon l'une des revendications précédentes, se présentant sous la forme de mascara, de rouge à lèvres, de brillant à lèvres (gloss), de fard à joues ou à paupières, de fond de teint.

15. Procédé de traitement cosmétique des matières kératiniques, notamment de la peau du corps ou du visage, des lèvres, des ongles, des cheveux et/ou des cils, comprenant l'application sur lesdites matières d'une composition cosmétique telle que définie à l'une des revendications 1 à 14.

16. Procédé selon la revendication précédente, **caractérisé en ce qu'**il s'agit d'un procédé cosmétique de soir ou de maquillage des lèvres.

17. Polycondensat susceptible d'être obtenu par réaction :
- de 10 à 30% en poids, par rapport au poids total du polycondensat, de pentaérythritol;
- de 45 à 80% en poids, par rapport au poids total du polycondensat, d'au moins un acide monocarboxylique non aromatique saturé, linéaire, ramifié et/ou cyclique, comprenant 6 à 32 atomes de carbone choisi parmi l'acide isostéarique seul ou l'acide stéarique seul ;
- de 0,1 à 10% en poids, par rapport au poids total du polycondensat, d'acide benzoïque;
- de 5 à 40% en poids, par rapport au poids total du polycondensat, d'acide isophtalique.

## Claims

1. Cosmetic or pharmaceutical composition comprising, in a cosmetically or pharmaceutically acceptable medium, at least one polycondensate that can be obtained by reaction:
- of 10 to 30 wt.%, relative to the total weight of the polycondensate, of pentaerythritol;
- of 30 to 80 wt.%, relative to the total weight of the polycondensate, of at least one saturated or unsaturated, linear, branched and/or cyclic, non-aromatic monocarboxylic acid, containing 6 to 32 carbon atoms chosen from isostearic acid alone or stearic acid alone;
- of 0.1 to 10 wt.%, relative to the total weight of the polycondensate, of benzoic acid;
- of 5 to 40 wt.%, relative to the total weight of the polycondensate, of isophthalic acid.

2. Composition according to the preceding claim, **characterized in that** the pentaerythritol represents 12 to 25 wt.%, and better still 14 to 22 wt.%, of the total weight of the polycondensate.

3. Composition according to either of the preceding claims, **characterized in that** the isostearic acid alone or the stearic acid alone represents 40 to 75 wt.%, or even 45 to 70 wt.%, and better still 50 to 65 wt.%, of the total weight of the final polycondensate.

4. Composition according to one of the preceding claims, **characterized in that** the benzoic acid represents 0.5 to 9.95 wt.%, better still 1 to 9.5 wt.%, or even 1.5 to 8 wt.%, of the total weight of the final polycondensate.

5. Composition according to one of the preceding claims, **characterized in that** the isophthalic acid represents 10 to 30 wt.%, and better still 14 to 25 wt.%, of the total weight of the polycondensate.

6. Composition according to one of the preceding claims, **characterized in that** the ratio of the number of moles of benzoic acid to the number of moles of isostearic acid alone or stearic acid alone is between 0.08 and 0.70, in particular between 0.10 and 0.60, or even between 0.12 and 0.40.

7. Composition according to one of the preceding claims, **characterized in that** the polycondensate has at least one of the following characteristics:
- an acid number, expressed in mg of potassium hydroxide per g of polycondensate, greater than or equal to 1; notably between 2 and 30, and better still between 2.5 and 15; and/or
- a hydroxyl number, expressed in mg of potassium hydroxide per g of polycondensate, greater than or equal to 40; notably between 40 and 120, and better still between 45 and 80; and/or
- a weight-average molecular weight (Mw) of between 1500 and 300 000, or even between 2000 and 200 000 and notably between 3000 and 100 000; and/or
- a viscosity, measured at 110°C, between 20 and 4000 mPa.s, notably between 30 and 3500 mPa.s, or even between 40 and 3000 mPa.s and better still between 50 and 2500 mPa.s; and/or
- a solubility in at least one solvent selected from isododecane, Parleam, isononyl isononanoate, octyldodecanol and C₁₂-C₁₅ alkyl benzoate, at the rate of at least 50 wt.%, at 70°C.

8. Composition according to one of the preceding claims, **characterized in that** the polycondensate is present in an amount between 0.1 and 70 wt.%, preferably between 1 and 50 wt.%, notably between 10 and 45 wt.%, or even between 20 and 40 wt.%, and better still between 25 and 35 wt.%, relative to the weight of the final cosmetic or pharmaceutical composition.

9. Composition according to one of the preceding claims, **characterized in that** the cosmetically or pharmaceutically acceptable medium comprises at least one oily liquid phase, which can contain at least one compound selected from the oils and/or solvents of mineral, animal, vegetable or synthetic origin, carbon-containing, hydrocarbon-containing, fluorinated and/or siliconized, volatile or non-volatile, alone or mixed.

10. Composition according to one of the preceding claims, **characterized in that** the cosmetically or pharmaceutically acceptable medium comprises at least one oil and/or solvent selected from, alone or mixed:
1/ esters of monocarboxylic acids with monoalcohols and polyalcohols; advantageously, said ester is a C12-C15 alkyl benzoate or corresponds to the following formula: R'₁-COO-R'2 where:
R'₁ represents a linear or branched alkyl radical with 1 to 40 carbon atoms, optionally having one or more ethylenic double bonds, optionally substituted and whose hydrocarbon chain can be interrupted by one or more heteroatoms selected from N and O and/or one or more carbonyl functions, and
R'₂ represents a linear or branched alkyl radical with 1 to 40 carbon atoms, optionally having one or more ethylenic double bonds, optionally substituted and whose hydrocarbon chain can be interrupted by one or more heteroatoms selected from N and O and/or one or more carbonyl functions;
2/ vegetable hydrocarbon oils with high content of triglycerides comprising esters of fatty acids and of glycerol, where the fatty acids can have chain lengths varying from C₄ to C₂₄, and the latter can be linear or branched, saturated or unsaturated;
3/ C₆-C₃₂, notably C₁₂-C₂₆, alcohols, and notably monoalcohols, such as oleic alcohol, linoleic alcohol, linolenic alcohol, isostearyl alcohol, 2-hexyldecanol, 2-butyloctanol, 2-undecylpentadecanol and octyldodecanol;
4/ volatile or non-volatile, linear or branched hydrocarbon oils, of synthetic or mineral origin, which can be selected from the hydrocarbon oils having from 5 to 100 carbon atoms, and notably petroleum jelly, polydecenes, hydrogenated polyisobutenes such as Parleam, squalane, perhydrosqualene, linear, branched and/or cyclic C₅-C₄₈ alkanes, and mixtures thereof;
5/ volatile or non-volatile silicone oils.

11. Composition according to one of the preceding claims, **characterized in that** the cosmetically or pharmaceutically acceptable medium comprises at least one oil and/or one solvent selected from, alone or mixed, isododecane, Parleam, isononyl isononanoate, octyldodecanol, phenyl trimethicone, C₁₂-C₁₅ alkyl benzoates and/or D5 (decamethylcyclopentasiloxane).

12. Composition according to one of the preceding claims, **characterized in that** the cosmetically or pharmaceutically acceptable medium comprises at least one compound selected from thickening agents, waxes, colouring matter, antioxidants, perfumes, essential oils, preservatives, cosmetic actives, hydrating agents, vitamins, ceramides, sun filters, surfactants, spreading agents, wetting agents, dispersants, antifoaming agents, neutralizing agents, stabilizers, polymers and notably fat-soluble film-forming polymers, and mixtures thereof.

13. Composition according to one of the preceding claims, being in the form of a product for care and/or make-up of the skin of the body or of the face, the lips, the eyelashes, the eyebrows, the hair, the scalp or the nails; a suntan product or self-tanning product; a hair-care product notably for the colouring, conditioning and/or care of the hair.

14. Composition according to one of the preceding claims, being in the form of mascara, lipstick, lip gloss, blusher, eye shadow, or foundation.

15. Method of cosmetic treatment of keratinous materials, notably of the skin of the body or of the face, the lips, the nails, the hair and/or the eyelashes, comprising the application of a cosmetic composition as defined in one of Claims 1 to 14 on said materials.

16. Method according to the preceding claim, **characterized in that** it is a cosmetic method for care or make-up of the lips.

17. Polycondensate that can be obtained by reaction:
- of 10 to 30 wt.%, relative to the total weight of the polycondensate, of pentaerythritol;
- of 45 to 80 wt.%, relative to the total weight of the polycondensate, of at least one saturated, linear, branched and/or cyclic non-aromatic monocarboxylic acid, containing 6 to 32 carbon atoms chosen from isostearic acid alone or stearic acid alone;
- of 0.1 to 10 wt.%, relative to the total weight of the polycondensate, of benzoic acid;
- of 5 to 40 wt.%, relative to the total weight of the polycondensate, of isophthalic acid.

## Patentansprüche

1. Kosmetische oder pharmazeutische Zusammensetzung, die in einem kosmetisch oder pharmazeutisch unbedenklichen Medium mindestens ein Polykondensat, das durch Reaktion von
- 10 bis 30 Gew.-%, bezogen auf die Gesamtmenge des Polykondensats, Pentaerythrit;
- 30 bis 80 Gew.-%, bezogen auf die Gesamtmenge des Polykondensats, mindestens einer gesättigten oder ungesättigten, linearen, verzweigten und/oder cyclischen nichtaromatischen Monocarbonsäure mit 6 bis 32 Kohlenstoffatomen, die aus Isostearinsäure alleine oder Stearinsäure alleine ausgewählt ist;
- 0,1 bis 10 Gew.-%, bezogen auf die Gesamtmenge des Polykondensats, Benzoesäure;
- 5 bis 40 Gew.-%, bezogen auf die Gesamtmenge des Polykondensats, Isophthalsäure
erhältlich ist, umfasst.

2. Zusammensetzung nach dem vorhergehenden Anspruch, wobei das Pentaerythrit 12 bis 25 Gew.-% und besser 14 bis 22 Gew.-% des Gesamtgewichts des Polykondensats ausmacht.

3. Zusammensetzung nach einem der vorhergehenden Ansprüche, wobei die Isostearinsäure alleine oder die Stearinsäure alleine 40 bis 75 Gew.-%, sogar 45 bis 70 Gew.-% und besser 50 bis 65 Gew.-% des Gesamtgewichts des fertigen Polykondensats ausmacht.

4. Zusammensetzung nach einem der vorhergehenden Ansprüche, wobei die Benzoesäure 0,5 bis 9,95 Gew.-%, besser 1 bis 9,5 Gew.-% oder sogar 1,5 bis 8 Gew.-% des Gesamtgewichts des fertigen Polykondensats ausmacht.

5. Zusammensetzung nach einem der vorhergehenden Ansprüche, wobei die Isophthalsäure 10 bis 30 Gew.-% und besser 14 bis 25 Gew.-% des Gesamtgewichts des Polykondensats ausmacht.

6. Zusammensetzung nach einem der vorhergehenden Ansprüche, wobei das Verhältnis zwischen der Molzahl von Benzoesäure und der Molzahl von Isostearinsäure alleine oder Stearinsäure alleine zwischen 0,08 und 0,70, insbesondere zwischen 0,10 und 0,60 oder sogar zwischen 0,12 und 0,40 liegt.

7. Zusammensetzung nach einem der vorhergehenden Ansprüche, wobei das Polykondensat mindestens eine der folgenden Eigenschaften aufweist:
- eine Säurezahl, ausgedrückt in mg Kaliumhydroxid pro g Polykondensat, größer gleich 1; insbesondere zwischen 2 und 30 und noch besser zwischen 2,5 und 15 und/oder
- eine Hydroxylzahl, ausgedrückt in mg Kaliumhydroxid pro g Polykondensat, größer gleich 40; insbesondere zwischen 40 und 120 und noch besser zwischen 45 und 80 und/oder
- ein gewichtsmittleres Molekulargewicht (Mw) zwischen 1500 und 300.000, sogar zwischen 2000 und 200.000 und insbesondere zwischen 3000 und 100.000 und/oder
- eine bei 110°C gemessene Viskosität zwischen 20 und 4000 mPa.s, insbesondere zwischen 30 und 3500 mPa.s, sogar zwischen 40 und 3000 mPa.s und noch besser zwischen 50 und 2500 mPa.s und/oder
- eine Löslichkeit in mindestens einem aus Isododecan, Parleam, Isononylisononanoat, Octyldodecanol und Benzoesäure-C₁₂-C₁₅-alkylester ausgewählten Lösungsmittel von mindestens 50 Gew.-% bei 70°C.

8. Zusammensetzung nach einem der vorhergehenden Ansprüche, wobei das Polykondensat in einer Menge zwischen 0,1 und 70 Gew.-%, vorzugsweise zwischen 1 und 50 Gew.-%, insbesondere zwischen 10 und 45 Gew.-%, sogar zwischen 20 und 40 Gew.-% und noch besser zwischen 25 und 35 Gew.-%, bezogen auf das Gewicht der fertigen kosmetischen oder pharmazeutischen Zusammensetzung, vorliegt.

9. Zusammensetzung nach einem der vorhergehenden Ansprüche, wobei das kosmetisch oder pharmazeutisch unbedenkliche Medium mindestens eine flüssige Fettphase umfasst, die mindestens eine aus Ölen und/oder Lösungsmitteln mineralischer, tierischer, pflanzlicher oder synthetischer Herkunft, die kohlenstoffhaltig, kohlenwasserstoffhaltig, fluorhaltig und/oder silikonhaltig sind und flüchtig oder nichtflüchtig sind, alleine oder im Gemisch ausgewählte Verbindung umfassen kann.

10. Zusammensetzung nach einem der vorhergehenden Ansprüche, wobei das kosmetisch oder pharmazeutisch unbedenkliche Medium mindestens ein Öl und/oder ein Lösungsmittel, das aus
1/Estern von Monocarbonsäuren mit Monoalkoholen und Polyalkoholen; wobei der Ester vorteilhafterweise ein Benzoesäure-C₁₂-C₁₅-alkylester ist oder der folgenden Formel entspricht: R'₁-C00-R'₂, wobei:
R'₁ für einen linearen oder verzweigten Alkylrest mit 1 bis 40 Kohlenstoffatomen, der gegebenenfalls eine oder mehrere ethylenische Doppelbindungen umfasst und gegebenenfalls substituiert ist und dessen Kohlenwasserstoffkette durch ein oder mehrere aus N und O ausgewählte Heteroatome und/oder eine oder mehrere Carbonylfunktionen unterbrochen sein kann, steht und
R'₂ für einen linearen oder verzweigten Alkylrest mit 1 bis 40 Kohlenstoffatomen, der gegebenenfalls eine oder mehrere ethylenische Doppelbindungen umfasst und gegebenenfalls substituiert ist und dessen Kohlenwasserstoffkette durch ein oder mehrere aus N und O ausgewählte Heteroatome und/oder eine oder mehrere Carbonylfunktionen unterbrochen sein kann, steht;
2/pflanzlichen Kohlenwasserstoffölen mit hohem Gehalt an Triglyceriden, die aus Estern von Fettsäuren und Glycerin, deren Fettsäuren Kettenlängen von C₄ bis C₂₄ aufweisen können, wobei letztere linear oder verzweigt und gesättigt oder ungesättigt sein können, bestehen;
3/C₆-C₃₂- und insbesondere C₁₂-C₂₆-Alkoholen und insbesondere -Monoalkoholen, wie Oleylalkohol, Linolylalkohol, Linolenylalkohol, Isostearylalkohol, 2-Hexyldecanol, 2-Butyloctanol, 2-Undecylpentadecanol und Octyldodecanol;
4/flüchtigen oder nichtflüchtigen, linearen oder verzweigten Kohlenwasserstoffölen synthetischer oder mineralischer Herkunft, die aus Kohlenwasserstoffölen mit 5 bis 100 Kohlenstoffatomen und insbesondere Vaseline, Polydecenen, hydrierten Polyisobutenen wie Parleam, Squalan, Perhydrosqualen, linearen, verzweigten und/oder cyclischen C₅-C₄₈-Alkanen und Mischungen davon ausgewählt sein können;
5/flüchtigen oder nichtflüchtigen Silikonölen alleine oder im Gemisch ausgewählt ist.

11. Zusammensetzung nach einem der vorhergehenden Ansprüche, wobei das kosmetisch oder pharmazeutisch unbedenkliche Medium mindestens ein Öl und/oder ein Lösungsmittel, das aus Isododecan, Parleam, Isononylisononanoat, Octyldodecanol, Phenyltrimethicon, Benzoesäure-C₁₂-C₁₅-alkylestern und/oder D5 (Decamethylcyclopentasiloxan) alleine oder im Gemisch ausgewählt ist.

12. Zusammensetzung nach einem der vorhergehenden Ansprüche, wobei das kosmetisch oder pharmazeutisch unbedenkliche Medium mindestens eine aus Verdickungsmitteln, Wachsen, Farbmitteln, Antioxidantien, Duftstoffen, etherischen Ölen, Konservierungsmitteln, kosmetischen Wirkstoffen, Feuchtigkeitsspendern, Vitaminen, Ceramiden, Sonnenschutzmitteln, Tensiden, Spreizmitteln, Netzmitteln, Dispergiermitteln, Antischaummitteln, Neutralisationsmitteln, Stabilisatoren, Polymeren und insbesondere fettlöslichen filmbildenden Polymeren und Mischungen davon ausgewählte Verbindung umfasst.

13. Zusammensetzung nach einem der vorhergehenden Ansprüche, die in Form eines Produkts zur Pflege und/oder zum Make-up der Körper- oder Gesichtshaut, der Lippen, der Wimpern, der Augenbrauen, der Haare, der Kopfhaut oder der Nägel, eines Sonnenschutz- oder Selbstbräunungsprodukts oder eines Haarprodukts, insbesondere zum Färben, Konditionieren und/oder Pflegen der Haare, vorliegt.

14. Zusammensetzung nach einem der vorhergehenden Ansprüche, die in Form eines Mascaras, eines Lippenstifts, eines Lip-Gloss, eines Rouges oder Lidschattens oder einer Grundierung vorliegt.

15. Verfahren zur kosmetischen Behandlung von Keratinmaterialien, insbesondere der Körper- oder Gesichtshaut, der Lippen, der Nägel, der Haare und/oder der Wimpern, bei dem man auf die Materialien eine kosmetische Zusammensetzung gemäß einem der Ansprüche 1 bis 14 aufbringt.

16. Verfahren nach dem vorhergehenden Anspruch, **dadurch gekennzeichnet, dass** es sich um ein kosmetisches Verfahren zur Pflege oder zum Make-up der Lippen handelt.

17. Polykondensat, das durch Reaktion von
- 10 bis 30 Gew.-%, bezogen auf die Gesamtmenge des Polykondensats, Pentaerythrit;
- 45 bis 80 Gew.-%, bezogen auf die Gesamtmenge des Polykondensats, mindestens einer gesättigten oder ungesättigten, linearen, verzweigten und/oder cyclischen nichtaromatischen Monocarbonsäure mit 6 bis 32 Kohlenstoffatomen, die aus Isostearinsäure alleine oder Stearinsäure alleine ausgewählt ist;
- 0,1 bis 10 Gew.-%, bezogen auf die Gesamtmenge des Polykondensats, Benzoesäure;
- 5 bis 40 Gew.-%, bezogen auf die Gesamtmenge des Polykondensats, Isophthalsäure
erhältlich ist.
